(19) Europäisches Patentamt
European Patent Office
Office européen des brevets

(11) **EP 3 362 023 B1**

(12) **EUROPÄISCHE PATENTSCHRIFT**

(45) Veröffentlichungstag und Bekanntmachung des Hinweises auf die Patenterteilung:
**13.07.2022  Patentblatt 2022/28**

(21) Anmeldenummer: **16800875.3**

(22) Anmeldetag: **12.10.2016**

(51) Internationale Patentklassifikation (IPC):
*A61H 1/02* *(2006.01)*      *A61B 5/00* *(2006.01)*

(52) Gemeinsame Patentklassifikation (CPC):
**A61H 1/0262; A61B 5/4528; A61B 5/486;**
**A61G 7/002; A61G 7/005; A61G 7/018;**
**A61H 1/0229; A61H 1/024; A61H 1/0244;**
A61B 5/1071; A61B 5/6891; A61B 2505/09;
A61H 2201/0142; A61H 2201/1215;
A61H 2201/1418;                          (Forts.)

(86) Internationale Anmeldenummer:
**PCT/DE2016/100475**

(87) Internationale Veröffentlichungsnummer:
**WO 2017/063639 (20.04.2017 Gazette 2017/16)**

(54) **REHABILITATIONSMECHANISMUS FÜR BETTPFLICHTIGE PATIENTEN**

REHABILITATION MECHANISM FOR PATIENTS CONFINED TO BED

MÉCANISME DE RÉÉDUCATION POUR PATIENTS ALITÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2015   DE 102015117435**
**15.10.2015   DE 102015117596**

(43) Veröffentlichungstag der Anmeldung:
**22.08.2018   Patentblatt 2018/34**

(73) Patentinhaber: **ReActive Robotics GmbH**
**80687 München (DE)**

(72) Erfinder:
• **KÖNIG, Alexander**
**80636 München (DE)**
• **SPIEGEL, Simon**
**81369 München (DE)**

(74) Vertreter: **Heilein, Ernst-Peter et al**
**HEILEIN IP LAW**
**Bezirksstraße 2**
**85716 Unterschleißheim (DE)**

(56) Entgegenhaltungen:
WO-A1-00/61059        JP-A- H10 258 101
JP-A- 2003 225 264    JP-A- 2005 334 385

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 362 023 B1

(52) Gemeinsame Patentklassifikation (CPC): (Forts.)
A61H 2201/1642; A61H 2201/50; A61H 2201/5061;
A61H 2201/5069

## Beschreibung

Die vorliegende Erfindung betrifft einen Rehabilitationsmechanismus für bettpflichtige Patienten sowie ein den Rehabilitationsmechanismus umfassendes Bett, insbesondere: Pflegebett, Krankenbett, Spitalbett oder Intensivbett.

Bei Menschen, welche beispielsweise an einer Krankheit oder den Folgen eines Unfalls leiden oder aus anderen Gründen für länger als die gewöhnlichen Nachtruhen als Patient an ein Bett "gefesselt" sind (im Folgenden als bettpflichtige Patienten bezeichnet), treten häufig Einschränkungen der Aktivität auf, welche dazu führen, dass solche Menschen nach Verlassen des Bettes schlecht bis gar nicht am sozialen Leben teilnehmen können, d.h. nicht oder nur bedingt arbeiten können und auf Hilfe im alltäglichen Leben angewiesen sind.

Durch Rehabilitation kann der Patient einen Teil seiner Aktivität wiedererlangen. In der Medizin werden unter Rehabilitation der Einsatz und die Wirkung von Maßnahmen verstanden, die darauf abzielen, die körperlichen, psychischen und sozialen Folgen einer Behinderung bzw. Aktivitätseinschränkung (engl. früher: *Disability,* jetzt: *Activity*) und Störung der Teilhabe (früher: *Handicap,* jetzt: *Participation*) am sozialen Leben auf ein Minimum zu beschränken.

Medizinische Rehabilitation ist nachgewiesenermaßen insbesondere für den menschlichen Stütz- und Bewegungsapparat von Bedeutung: werden nämlich Knochen, Gelenke, Muskeln und Sehnen insbesondere der menschlichen Beine (bestehend aus Gesäß, Hüftgelenk, Oberschenkel, Kniegelenk, Unterschenkel und Fuß) nicht regelmäßig bewegt, versteifen sie - die diesbezüglichen, im menschlichen Rückenmark angesiedelten, Lokomotionszentren können verkümmern.

Anders als Menschen, die körperlich und aufgrund einer stabilen Kreislaufsituation in der Lage sind, beispielsweise an einem Laufbandtraining teilzunehmen, ist dieses bettpflichtigen Patienten für gewöhnlich verwehrt. Ursachen können insbesondere orthopädische, intensivmedizinische und/oder neurologische Aktivitätseinschränkungen sein, welche einzeln oder kumulativ angetroffen werden können.

Rehabilitation orthopädischer Aktivitätseinschränkungen

Die Orthopädie ist das Tätigkeitsfeld des Facharztes für Orthopädie und Unfallchirurgie, das sich mit der Entstehung, Verhütung, Erkennung und Behandlung angeborener oder erworbener Form- oder Funktionsfehler des Stütz- und Bewegungsapparates, also der Knochen, Gelenke, Muskeln und Sehnen, sowie mit der Rehabilitation solcher Patienten befasst.

Orthopädische Behandlungen bedienen sich unter anderem chirurgischer Verfahren wie insbesondere der Prothesenchirurgie (z.B., aber nicht limitiert auf, Hüft- oder Kniegelenksersatz). Nach einem Unfall oder chirurgischen Eingriff ist bettpflichtigen Patienten aufgrund orthopädischer Aktivitätseinschränkungen für gewöhnlich eine vollständige Belastung der Knochen, Gelenke, Muskeln und Sehnen eines oder beider Beine mit dem eigenen Körpergewicht nicht möglich.

Um dennoch ein Versteifen der Beine zu vermeiden ist beispielsweise aus der WO 00/45897 A1 eine Im-Bett-Übungsmaschine bekannt geworden, welche endseitig an ein Pflege- oder Krankenbett herangeschoben in liegender Position die Durchführung von zyklischen Bein-Bewegungen gestattet. Die bekannte Im-Bett-Übungsmaschine gestattet jedoch insbesondere keine Übungen in einer vertikalisierten Position. Damit aber die Füße ganz oder teilweise mit dem Druck des eigenen Körpergewichts belastet werden, was den Heilungsprozess, z.B. nach Gelenkersatz oder Knochenbruch, beschleunigen kann, ist es notwendig, den bettpflichtigen Patienten ganz oder teilweise in eine vertikalisierte Lage bringen zu können.

Diesem Aspekt trägt beispielsweise der aus der WO 00/61059 A1 bekannt gewordene Stehtisch Rechnung. Problematisch dabei ist, dass dieser Stehtisch oder dazu vergleichbare bekannte Vorrichtungen gewöhnlich in einem separaten Trainingsraum stehen, in jedem Fall aber eine Umlagerung des Patienten vom Bett auf die jeweilige Rehabilitationsvorrichtung erfordern. Dies ist zumindest bei intensivpflichtigen, also auf intensivmedizinische Leistungen angewiesene, Patienten in der Regel ausgeschlossen, in jedem Fall aber mit besonderen Risiken verbunden.

Rehabilitation intensivmedizinischer Aktivitätseinschränkungen

Die Intensivmedizin ist ein medizinisches Fachgebiet, das sich mit Diagnostik und Therapie lebensbedrohlicher Zustände und Krankheiten befasst. Das Erbringen intensivmedizinischer Leistungen geschieht meist in besonders ausgerüsteten Stationen eines Krankenhauses bzw. Spitals, den sog. Intensivstationen, die durch speziell ausgebildete Fachärzte, wie Anästhesisten, Internisten, Chirurgen oder Neurologen, geführt werden.

Die Ergebnisse intensivmedizinischer Leistungen umfassen entsprechend der grundlegenden Erkrankung eine große Spannweite. Prinzipiell muss eine gewisse günstige Prognose des krankhaften Zustandes gegeben sein. Ziel intensivmedizinischer Leistungen nämlich ist die Wiederherstellung der völligen Gesundheit oder wenigstens das Erreichen eines weitgehend autonomen Zustandes des Patienten. Sogenannte lebensverlängernde Maßnahmen verfolgen somit keinen Selbstzweck.

Auf Intensivstationen werden Patienten aufgenommen, deren Zustand lebensbedrohlich ist oder deren Zustand lebensbedrohlich werden könnte, insbesondere bedingt durch ein labiles Herz-Kreislauf-System, Risiko von Herzstillstand, Infektionsrisiken und dergleichen mehr. Dieser Tatsache tragen auf Intensivstationen standardisierte Überwachungsmaßnahmen Rechnung.

Intensivstationen sind baulich und gerätetechnisch aufwendig ausgestattet. Einen Schwerpunkt bildet dabei die Gestaltung des Intensivbettes, welches der sicheren Lagerung Schwerstkranker auf Intensivstationen dient. Neben einer die Überwachungsmaßnahmen unterstützenden Apparatur zeichnet sich ein Intensivbett insbesondere durch eine Matratze aus geeignet ausgebildet sowohl zur Vorbeugung von Dekubitus als auch für eine sofortige manuelle Reanimation zumindest von Herz- und/oder Lunge eines intensivpflichtigen Patienten. Ferner muss die Matratze zur Durchführung von Defibrillationen nichtleitend und beständig gegen Flüssigkeiten, Blut sowie Wischdesinfektionen mit handelsüblichen Desinfektionsmitteln sein. Um intensivpflichtige Patienten gegen ein Herausfallen zu sichern ist die Matratze gewöhnlich von Längs- und Querseitenbegrenzungen umgeben, welche an den Längs- bzw. Querseiten eines Bett- oder Matratzenrahmens befestigbar sind und nicht selten zumindest einen Teil der Überwachungsapparatur haltern.

Aufgrund dieser typischen Gestaltung von Intensivbetten können die bekannten, oben beschriebenen Rehabilitationsvorrichtungen nicht einfach an ein modernes Intensivbett herangeführt werden und/oder erfordern eine Umlagerung des Patienten. Letzteres aber - darauf wurde schon hingewiesen - ist bei intensivpflichtigen, normalerweise labilen oder aus anderen Gründen auf intensivmedizinische Leistungen angewiesene, Patienten in der Regel ausgeschlossen, in jedem Fall aber mit besonderen Risiken verbunden.

Dabei tragen Patienten intensivmedizinischer Einrichtungen ohnehin bereits ein fünf- bis zehnfach höheres Infektionsrisiko gegenüber Patienten von Normalstationen. So addieren sich bei intensivpflichtigen Patienten verschiedene infektionsbegünstigende Faktoren, die sowohl vom Patienten selbst als auch von intensivmedizinisch angewandten Behandlungsmaßnahmen (viele Katheter, Schläuche, etc .... ) ausgehen können. Deshalb sind, um das Infektionsrisiko zu verringern, auf Intensivstationen besondere Hygienemaßnahmen vorgeschrieben, denen Rehabilitationsvorrichtungen wie die bekannte Im-Bett-Übungsmaschine oder der bekannte Stehtisch nur schwerlich genügen.

Mit Rehabilitationsvorrichtungen unterstützte Rehabilitationsstrategien finden daher bislang für gewöhnlich erst Anwendung, nachdem der Patient die Intensivstation verlassen hat.

Rehabilitation neurologischer Aktivitätseinschränkungen

Die Neurologie ist die Lehre von den Erkrankungen des Nervensystems. Die Organsysteme, die in der Neurologie Berücksichtigung finden, sind das Zentralnervensystem, also Gehirn und Rückenmark, seine Umgebungsstrukturen und blutversorgende Gefäße sowie das periphere Nervensystem einschließlich dessen Verbindungsstrukturen mit den Muskeln sowie die Muskulatur.

In der neurologischen Rehabilitation haben neueste Studien gezeigt, dass Rehabilitation möglichst früh beginnen sollte. Um den Rehabilitationserfolg zu maximieren sollte beispielsweise bereits 24 Stunden nach einem Schlaganfall mit Querschnitts- oder anderen Lähmung oder einer traumatischen Hirnschädigung mit oder ohne einer quantitativen Bewusstseinsstörung, welche in ihrer schwersten Form ein Koma darstellt, mit Rehabilitations-Maßnahmen begonnen werden.

Da sich von Lähmung und/oder Bewusstseinsstörung betroffene Patienten zu diesem Zeitpunkt für gewöhnlich noch auf einer Intensivstation befinden, gestalten sich frühzeitig aufzunehmende neurologische Rehabilitationsstrategien als doppelt schwierig: neben der schon beschriebenen, nicht unproblematischen Intensivumgebung müsste nämlich zumindest bei gelähmten und/oder komatösen Patienten die gesamte Beinbewegung zumindest anfänglich allein von der Rehabilitationsvorrichtung zyklisch durchgeführt werden.

In diesem Zusammenhang insbesondere problematisch ist, dass die Position des Patienten im Bett in der Regel unbestimmt ist. Um den Kontakt zwischen Patient und Rehabilitationsvorrichtung herzustellen kann entweder der Patient solange bewegt werden, bis geeignete Gliedmaße insbesondere seines Beines mit geeigneten Kontaktpunkten einer Rehabilitationsvorrichtung übereinstimmen, oder es kann versucht werden, die Rehabilitationsvorrichtung in ihrer Lage zum Patienten so lange zu verstellen, dass eine hinreichende Übereinstimmung erreicht ist. Beide beschriebenen Szenarien benötigen Zeit und sind zudem fehleranfällig, da die Übereinstimmungen gewöhnlich manuell durchgeführt und insoweit nicht perfekt erreicht sein müssen.

In diesem Zusammenhang offenbart die JP 2005 334385 A einen seitlichen Kontaktpunkt eines Arms mit einem Drehgelenk mit entsprechendem Krafteintrag. Des Weiteren offenbart die JP H10 258101 A eine seitliche Kontaktierung und Krafteintrag über ein oder zwei Kontaktstellen an Ober- bzw. Unterschenkel bzw. eine Kontaktierung und Krafteintrag des Unterschenkels von unten. Schließlich offenbart die JP 2003 225264 A, dass lediglich der Unterschenkel sowie das Sprunggelenk eines Patienten mittels eines Rehabilitationsmechanismus kontaktiert wird. Darüber hinaus erfolgt auch bei der JP 2003 225264 A die Kontaktierung wiederrum seitlich bzw. von unten.

Weiterhin ist folgendes Problem anzutreffen: Beginnt eine Therapie mittels einer Rehabilitationsvorrichtung, so kann es regelmäßig vorkommen, dass der Patient verrutscht, beispielsweise wenn das Bett ganz oder teilweise vertikalisiert wird und Gravitationskräfte den Patienten nach unten ziehen. Zuvor eingestellte Kontaktpunkte zwischen Rehabilitationsvorrichtung und geeigneten Gliedmaßen, insbesondere der Beine eines Patienten, lägen zueinander nicht mehr optimal. Um die Kontaktpunkte neu zu justieren muss die Rehabilitationsvorrichtung angehalten und neu ausgerichtet werden, bevor eine Therapie dann fortgesetzt werden kann.

Aus diesen Gründen finden in der Praxis bislang Übungen zur Aktivhaltung von Patienten intensivmedizinischer Einrichtungen allenfalls durch spezielle Physiotherapeuten statt, welche die Gliedmaßen intensivpflichtiger Patienten - soweit möglich täglich, mindestens jedoch mehrmals wöchentlich - manuell von Hand bewegen. Diese manuelle Physiotherapie hat nun zum Nachteil, dass die Therapeuten aufgrund der physischen Anstrengungen schnell ermüden können, was zu kaum planbaren, geschweige denn auswertbaren, Anwendungsfortschritten führt. Weiterhin ist nicht garantiert, dass der Physiotherapeut bei jeder Physiotherapieanwendung mit gleichem (maximalen) Einsatz und Effizienz arbeitet. Auch kann der Physiotherapeut keine objektive, sondern nur eine subjektive Quantifizierung der Aktivität des Patienten durchführen, was die objektive Quantifizierung des Therapieerfolges über mehrere Therapieanwendungen erschwert. Schließlich kann, besonders im intensivmedizinischen Umfeld, eine Therapieanwendung nicht nur die Anwesenheit eines oder mehrerer Physiotherapeuten erfordern, sondern ebenfalls die Anwesenheit der Krankenschwester/des Krankenpflegers, welche/r die Vitalparameter des Patienten während der Anwendung überwachen muss um z.B. auf Herz-Kreislauf Probleme reagieren zu können. Durch die zusätzliche Anwesenheit von hochqualifiziertem Klinikpersonal werden derartige Therapieanwendungen kaum bezahlbar.

Hiervon ausgehend liegt der vorliegenden Erfindung die Aufgabe zugrunde, einen gegenüber dem Stand der Technik verbesserten Rehabilitationsmechanismus, insbesondere für aufgrund orthopädischer, intensivmedizinischer und/oder neurologischer Aktivitätseinschränkungen bettpflichtig gewordener Patienten, bereitzustellen, welcher ohne den Patienten umbetten zu müssen eine planmäßig automatisierte Rehabilitation zumindest der Gelenke, Muskeln und Sehnen der Beine bettpflichtiger Patienten gestattet. Darüber hinaus soll ein bevorzugt ausgebildeter Rehabilitationsmechanismus nach der Erfindung ganz oder teilweise automatisch auf veränderte Kontaktpunkte zwischen Rehabilitationsmechanismus und geeigneten Gliedmaßen, insbesondere der Beine eines Patienten, adjustieren können. Neben handelsüblichen oder eigens geschaffenen Pflege- oder Krankenbetten schließlich soll der Rehabilitationsmechanismus insbesondere auch in handelsüblichen oder eigens geschaffenen Spital- oder Intensivbetten einsetzbar sein, und zwar unabhängig davon, ob der bettpflichtige Patient in dem jeweiligen Bett ganz oder teilweise in eine vertikalisierte Lage gebracht werden kann, wobei in einer jeden vom bettpflichtigen Patienten zwischen einer horizontal und einer vertikalisiert eingenommenen Lage der Rehabilitationsmechanismus eine rhythmische Be- und Entlastung der Fußsohlen bettpflichtiger Patienten unterstützen können soll.

Diese Aufgabe wird zunächst durch einen Rehabilitationsmechanismus mit den Merkmalen des unabhängigen Patentanspruchs 1 gelöst.

Ein Rehabilitationsmechanismus nach der Erfindung für ein Bett mit sich in y-Richtung erstreckenden Längsseiten sowie in x-Richtung erstreckenden Querseiten, welcher für eine planmäßig automatisierte Rehabilitation zumindest der Gelenke, Muskeln und Sehnen der Beine eines bettpflichtigen Patienten geeignet ausgebildet ist, umfasst wenigstens:

- ein in Wirkverbindung mit den Kniegelenken des pflichtigen Patienten bringbares Kniemodul;

- wenigstens einen Winkelsensor, welcher den von einem Verbindungselement zur Knie-Orthese und/oder zum Ausleger eingenommenen Winkel überwacht; und

- ein Steuermodul zur Erzielung planmäßiger Rehabilitationsbewegungen zumindest der Gelenke, Muskeln und Sehnen der Beine des bettpflichtigen Patienten mittels des Kniemoduls,

- wobei das Steuermodul eingerichtet ist, bei Über- oder Unterschreitung einer variabel vorgegebenen, durch den Winkelsensor überwachten, Soll-Winkelschwelle $\varphi\_soll$ eine Mechanik dergestalt anzusteuern, dass ein Ist-Winkel $\varphi\_ist$ durch Adjustierung der Exzenterscheiben unterhalb der überschrittenen oder oberhalb der unterschrittenen Schwellen auf die Soll-Winkelschwelle $\varphi\_soll$ zurückgeführt wird.

Er zeichnet sich zusätzlich dadurch aus, dass das Kniemodul wenigstens umfasst:

- eine in z-Richtung oberhalb einer Matratze angeordnete Knie-Orthese zur Aufnahme eines Kniegelenkes des bettpflichtigen Patienten bei Benutzung, wobei das Kniegelenk dabei stets auf Höhe eines zentralen Gelenkes der Knie-Orthese ist;

- ein mit der Knie-Orthese verbundenes Verbindungselement;

- einen in z-Richtung oberhalb von Patient und Matratze angeordneten proximalen Abschnitt eines Auslegers, an welchem das Verbindungselement befestigt ist,

- und zur unmittelbaren oder mittelbaren Abstützung an einem Bett- oder Matratzenrahmen eine mittels des Steuermoduls ansteuerbare Mechanik, welche über

- den proximalen Abschnitt des Auslegers,
- das Verbindungselement und
- einen Kontaktpunkt, an dem der proximale Abschnitt des Auslegers mit dem Verbindungselement verbunden ist, und welcher genau oberhalb des Drehpunktes der Knie-Orthese liegt,

eine definierte Kraft dergestalt in z-Richtung genau oberhalb eines Drehpunkts der Knie-Orthese in diese einleitet, dass über das darin bei Benutzung aufgenommene Kniegelenk des bettpflichtigen Patienten die Gelenke, Muskeln und Sehnen dieses Beines planmäßige Rehabilitationsbewegungen ausführen,

- wobei

  - die Mechanik zwei Exzenter umfasst, welche jeweils durch eine auf einer Exzenterwelle angebrachten Exzenterscheibe, deren Mittelpunkt jeweils außerhalb der Wellenachse liegt, gebildet sind;
  - die Exzenterwellen durch einen Elektromotor angetrieben sind; und
  - ein distaler Abschnitt des Auslegers mit einem Steuerbolzen der ersten Exzenterscheibe über ein Radiallager und mit einem Steuerbolzen der zweiten Exzenterscheibe über ein Gleitlager wirkverbunden ist,

- und wobei das Steuermodul eingerichtet ist, die Soll-Winkelschwelle $\varphi\_\text{soll}$ in Abhängigkeit von den Exzenterwinkeln alpha und beta immer so zu bestimmen, dass das Verbindungselement zwischen Ausleger und Orthese stets senkrecht auf der Matratze steht.

Die modulare Ausbildung des Rehabilitationsmechanismus hat zum Vorteil, dass bett- und insbesondere intensivpflichtige Patienten eine planmäßig automatisierte Rehabilitation unmittelbar in ihrem Bett erhalten können und zwar auch ohne riskante Umbettung und/oder Möglichkeiten zu einem Eigenbeitrag.

Unter dem Begriff "Modul" bzw. "modulare Ausbildung" soll im Folgenden insbesondere verstanden werden, dass die so bezeichneten Bauteile einzelne, in sich abgeschlossene Baugruppen bilden, die zwar mit weiteren Elementen in Wirkbeziehung stehen, von diesen aber reversibel zum Zwecke der Verstauung und/oder zum Transport trennbar sind.

Durch die Anordnung einzelner Bauteile eines Kniemoduls in z-Richtung oberhalb einer Matratze bzw. oberhalb von Patient und Matratze und dessen Abstützung unmittelbar oder mittelbar an einem Bett- oder Matratzenrahmen ist es insbesondere ermöglicht, auf die Kniegelenke bettpflichtiger Patienten eine, dessen Fußsohlen vorteilhaft rhythmisch be- und entlastende, Unterstützungskraft aufzubringen und zwar in einer jeden vom Patienten zwischen einer horizontal und einer vertikalisiert eingenommenen Lage.

Bei orthopädischen Patienten ist die rhythmische Be- und Entlastung der Fußsohlen wichtig, um z.B.

ein verletztes Gelenk wieder an das Gehen und/oder bei einer ganz oder teilweise vertikalisiert eingenommenen Lage an eine Belastung zu gewöhnen.

Bei intensivpflichtigen Patienten ist die rhythmische Be- und Entlastung der Fußsohlen von Bedeutung, um einem Versteifen der Beine und einem Verkümmern der im Rückenmark angesiedelten Lokomotionszentren vorzubeugen.

Bei neurologischen Patienten erzeugt die alternierende Bewegung in den Fußsohlen zusätzlichen sensorischen Input, der an das zentrale Nervensystem weitergeleitet wird. Dieser sog. "afferente sensorische Input" sorgt dafür, dass auch die Hirnregionen angeregt werden, welche in der Generierung von Gehbewegungen involviert sind.

Im Lichte des vorstehenden ist deshalb in einer Weiterbildung ein Rehabilitationsmechanismus bevorzugt, welcher weiterhin ein in Wirkverbindung mit den Füßen und/oder insbesondere den Fußsohlen des bettpflichtigen Patienten bringbares Fußmodul umfasst.

Die Module, insbesondere das Kniemodul oder auch ein etwaiges Fußmodul sind mechanisch und elektrisch vollständig von einem Bett, an dem diese eingesetzt werden, trennbar und entfernbar und so separat verstaubar. Alternativ oder zusätzlich können diese Module weggeklappt werden, etwa in einen Raum unterhalb einer Matratze eines Bettes.

Die Modularität/Abnehmbarkeit/Entfernbarkeit des Kniemoduls und ein etwaiges Fußmodul vom Bett, sei es durch Abtrennung oder durch Verstauung der Therapiemodule unter dem Bett, ist besonders vorteilhaft, da so das Bett außerhalb der Therapiezeiten als normales Bett genutzt werden kann. Unter "normaler Nutzung" ist hierbei zu verstehen, dass kein Element des Rehabilitationsmechanismus den Zugang zum Patienten von allen Seiten in irgendeiner Form behindert, den Transfer von/in das Bett behindern oder evtl. notwendige Notmaßnahmen oder Pflegemaßnahmen behindert oder erschwert.

Zum therapeutischen Kontext der vorliegenden Erfindung sei an dieser Stelle auch auf die WO 2015/158664 A1 der Anmelderin verwiesen, auf welche vorsorglich, insbesondere auch hinsichtlich der dort beschriebenen Fußmodul-Varianten, vollumfänglich Bezug genommen wird.

Gegenstand der vorliegenden Erfindung ist daher auch ein den erfindungsgemäßen Rehabilitationsmechanismus umfassendes Bett, wobei dieses z.B. als handelsübliches oder eigens gebautes Pflegebett, Krankenbett, Spitalbett oder insbesondere als Intensivbett ausgebildet sein kann.

Bevorzugt ist der Rehabilitationsmechanismus reversibel lösbar an einem Krankenhausbett, insbesondere einem herkömmlichen Krankenhausbett, festsetzbar. Er ist dazu vorgesehen und eingerichtet an herkömmlichen Krankenhausbetten reversibel angeordnet zu werden, um so einen im Bett liegenden Patienten einer Therapie zu unterziehen. Das weist der Rehabilitationsmechanismus vorzugsweise Stütz- und/oder Klemmmittel auf, um die reversible Festsetzbarkeit zu erreichen. Vorzugsweise ist der Rehabilitationsmechanismus als Modul von einem Krankenhausbett, insbesondere einem herkömmlichen Krankenhausbett, abnehmbar und/oder unter dem Krankenhausbett verstaubar. Hierdurch wird die Verwendung des Rehabilitationsmechanismus wesentlich vereinfacht. Er stellt ein in sich geschlossenes System dar, welches wahlweise an bestehenden Krankenhausbetten einsetzbar ist.

Die mit einem Rehabilitationsmechanismus nach der Erfindung ermöglichte planmäßig automatisierte Rehabilitation zumindest der Gelenke, Muskeln und Sehnen der Beine bett- und insbesondere intensivpflichtiger Patienten hat zum Ziel, Aktivitätseinschränkungen und/oder Störung der Teilhabe am sozialen Leben auf ein Minimum zu beschränken. Die zentrale therapeutische Idee dahinter ist, die Aktivität bett- oder insbesondere intensivpflichtiger Patienten frühestmöglich, also noch im Bett zu quantifizieren und/oder auf ein gewünschtes Niveau zu regeln. Die diesbezüglich im Rahmen der vorliegenden Erfindung erforderliche Bestimmung der einzelnen Parameter einer planmäßig automatisierten Rehabilitation verantworten gleichwohl in der Praxis Physiotherapeuten oder mindestens vergleichbar ausgebildete Fachkräfte.

Besonders bevorzugt ist die Rehabilitationsbewegung eine Gehbewegung, Schrittbewegung und/oder Treppensteigen-simulierende Bewegung. Eine Gehbewegung, Schrittbewegung oder Treppensteigen-simulierende Bewegung ist wesentlich vorteilhafter bei der Rehabilitation als beispielsweise eine Fahrradfahrbewegung. Aus DE 41 13 135 A1 ist beispielsweise ein reines Fußmodul bekannt, welches eine Fahrradfahrbewegung erlaubt. Allerdings ist es für Rehabilitationspatienten weitaus wichtiger, eine Gehbewegung, Schrittbewegung oder Treppensteigen-simulierende Bewegung zu erlernen und die Belastung, die bei einer solchen Gehbewegung, Schrittbewegung oder Treppensteigen-simulierende Bewegung auftritt, zu simulieren und die Fortschritte hierbei zu messen. Eine Fahrradfahrbewegung eignet sich nur unter bestimmten Bedingungen, da hier insbesondere kein Abrollen des Fußes auftritt und auch ein Moment, das auf das Sprunggelenk des Fußes ausgeübt wird, tendenziell gering ist.

Gemäß einer bevorzugten Weiterbildung der Erfindung bilden das Fußmodul und das Kniemodul gemeinsam ein Exoskelett für den Patienten. Die das Exoskelett bildenden Module wirken mittels des Steuermoduls zusammen und unterstützen den Patienten bei der Ausübung der Bewegung.

Weiterhin umfasst der Rehabilitationsmechanismus bevorzugt ein Biofeedbackmodul zum Bereitstellen eines visuellen und/oder auditiven Feedbacks für den Patienten. Ein solches Biofeedbackmodul weist bevorzugt ein Display oder dergleichen auf, das im Sichtfeld des Patienten angeordnet wird, um diesem Feedback zu geben. Ein solches Biofeedbackmodul kann grundsätzlich wie in US 2010/0042022 A1 offenbart ausgebildet sein. Es ist bevorzugt dazu ausgebildet, dem Patienten anzuzeigen, wenn dieser eine Bewegung korrekt ausführt und/oder Fortschritte macht. Ferner ist das Biofeedbackmodul vorzugsweise dazu ausgebildet, dem Patienten anzuzeigen, wenn dieser eine Bewegung nicht korrekt ausführt, eine Übung wechseln sollte, die Übung beenden sollte und dergleichen.

Weitere vorteilhafte Ausgestaltungen und Weiterbildungen, welche einzeln oder in Kombination miteinander einsetzbar sind, sind Gegenstand der abhängigen Ansprüche.

Diese sowie zusätzliche Einzelheiten und weitere Vorteile der Erfindung werden nachfolgend an Hand bevorzugter Ausführungsbeispiele, auf welche die vorliegende Erfindung jedoch nicht beschränkt ist, und in Verbindung mit der beigefügten Zeichnung beschrieben.

Darin zeigen schematisch:

Fig. 1     einen bett- oder intensivpflichtigen Patienten in einem handelsüblichen Bett nach dem Stand der Technik, insb. Intensivbett, mit Längs- und Querseitenbegrenzungen wie insb. einer fußseitig oder kopfseitig angeordneten Begrenzungsplatte in einer perspektivischen Ansicht;

Fig. 2     das Bett aus Fig. 1 mit einem fußseitig an Stelle einer handelsüblichen Begrenzungsplatte oder wie dargestellt mittels einer speziell statisch ausgelegten Tragplatte angeordnetem Rehabilitationsmechanismus;

Fig. 3     einen Ausschnitt des in Fig. 2 gezeigten Rehabilitationsmechanismus in einer perspektivischen Ansicht;

Fig. 4     das Bett aus Fig. 2 mit einem am Bett gesicherten Patienten und mit angelegten Knie- und Fußmodul eines Rehabilitationsmechanismus vor einer Vertikalisierung des Bettes in einer perspektivischen Seitenansicht;

Fig. 5     das Bett aus Fig. 4 in einem vertikalisierten Zustand;

Fig. 6     den Rehabilitationsmechanismus nach der Erfindung aus Fig. 5 in einer vergrößerten perspektivischen Ansicht;

Fig. 7     den Rehabilitationsmechanismus aus Fig. 6 in einer Vorderansicht;

Fig. 8a     bis c bzw. 10a bis 10c eine automatisierte Adjustierung nach Veränderung der Kontaktpunkte zwischen dem Rehabilitationsmechanismus und den Gliedmaßen eines Patienten mit langen (Fig. 8a bis c) bzw. mit kurzen Beinen (Fig. 10a bis c);

Fig. 9a   bis c bzw. 11a bis c die von dem Rehabilitationsmechanismus über die Knie-Orthese erzeugten Trajektorien (T) zum Beugen bzw. Strecken der Beine eines Patienten langen (Fig. 9a bis c) bzw. mit kurzen Beinen (Fig. 11a bis c);

Fig. 12   beispielhaft anhand des Auslegers aus Fig. 8c in einer vergrößerten Darstellung, wie mittels wenigstens eines Winkelsensors der vom Verbindungselement zur Knie-Orthese und/oder zum Ausleger eingenommene Winkel phi (φ) überwacht werden kann;

Fig. 13   wie abweichend von einer Sollwinkelstellung (φ_soll) das Kniegelenk eines Patienten in Richtung des Fußendes des Bettes verrutscht sein kann;

Fig. 14   wie abweichend von einer Soll-Winkelstellung (φ_soll) das Kniegelenk eines Patienten in Richtung des Kopfende des Bettes verrutscht sein kann;

Fig. 15   anhand einer Prinzipskizze einen Regelkreis zur Erzeugung einer planmäßigen Rehabilitationsbewegung mittels eines Rehabilitationsmechanismus; und

Fig. 16   anhand einer Prinzipskizze einen gegenüber Fig. 15 erweiterten Regelkreis zur Erzeugung einer sequentiellen oder zeitgleich zu planmäßigen Rehabilitationsbewegungen erfolgenden Adjustierung der Exzenter eines Rehabilitationsmechanismus.

Bei der nachfolgenden Beschreibung bevorzugter Ausführungsformen der vorliegenden Erfindung bezeichnen gleiche Bezugszeichen gleiche oder vergleichbare Komponenten.

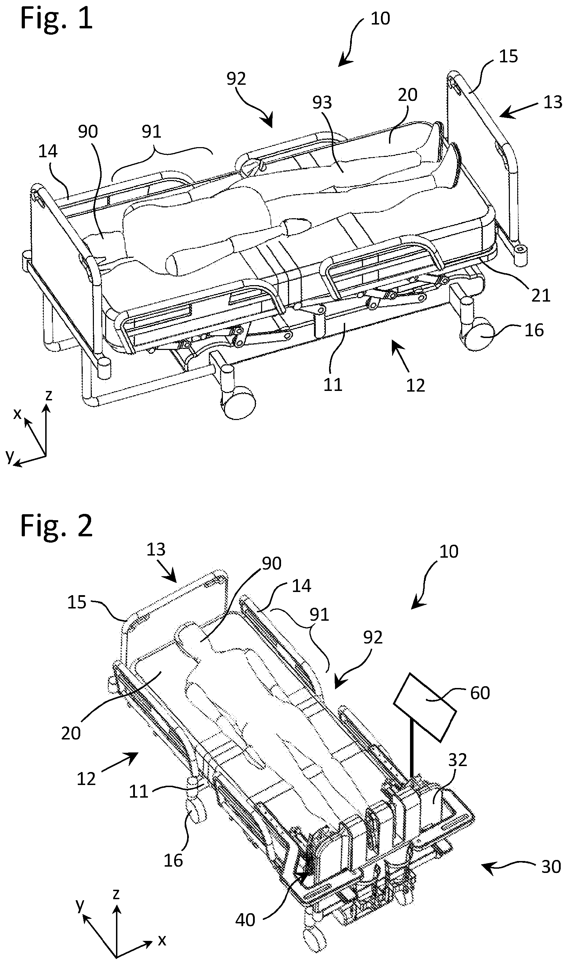

Fig. 1 zeigt einen bett- oder intensivpflichtigen Patienten 90 in einem handelsüblichen Bett 10 nach dem Stand der Technik, insb. Intensivbett, in einer perspektivischen Ansicht. Das dargestellte Bett 10 kann insbesondere für typischerweise an Intensivbetten gestellten medizinischen Anforderungen ausgebildet, aber auch in einem Nicht-Intensiv Umfeld eingesetzt sein, insbesondere als Pflege-, Kranken- oder Spitalbett. Neben einer die Überwachungsmaßnahmen unterstützenden Apparatur (nicht dargestellt), zeichnet sich das dargestellte Bett 10 durch eine Matratze 20 aus geeignet ausgebildet zumindest zur Vorbeugung von Dekubitus. Im Fall eines Intensivbettes zeichnet sich das dargestellte Bett 10 zudem durch eine Matratze 20 aus, welche zusätzlich für eine sofortige manuelle Reanimation zumindest von Herz und/oder Lunge 91 eines intensivpflichtigen Patienten 90 ausgebildet und zur Durchführung von Defibrillationen nichtleitend und beständig ist gegen Flüssigkeiten, Blut sowie Wischdesinfektionen mit handelsüblichen Desinfektionsmitteln, wobei aus Reinigungs- bzw. Desinfektionsgründen ungeteilte bzw. durchgängig ausgebildete Matratzen 20 bevorzugt sind. Um bett- und insbesondere intensivpflichtige Patienten 90 gegen ein Herausfallen zu sichern, ist die Matratze 20 ganz oder teilweise von Längs- 14 und Querseitenbegrenzungen 15 umgeben, welche beispielsweise an Längs- 12 bzw. Querseiten 13 eines Bett- 11 oder Matratzenrahmens 21 des Bettes 10 befestigbar sind, die für gewöhnlich zumindest einen Teil der Überwachungsapparatur haltern (nicht dargestellt). Um das Bett 10 bewegen zu können, weist dieses beispielsweise Rollbeine 16 auf. Zur Verbesserung der Manövrierbarkeit, können die Rollbeine 16 motorgetrieben ausgebildet sein. Bevorzugt sind auch Ausgestaltungen, bei denen der Bett- 11 und/oder Matratzenrahmen 21 höhen- und/oder in der Neigung (gleich ob längs oder quer) verstellbar ausgebildet sind, wobei das Kopf- und Fußende vorzugsweise separat, also mit unterschiedlichen und/oder entgegengesetzten Neigungen zueinander, verstellbar ausgebildet sein können (nicht dargestellt).

Fig. 2 zeigt das Bett 10 aus Fig. 1 mit einem fußseitig an Stelle einer handelsüblichen Begrenzungsplatte oder - wie dargestellt - mittels einer speziell statisch ausgelegten Tragplatte 32 angeordnetem Rehabilitationsmechanismus 30. Erkennbar ist, wie der Rehabilitationsmechanismus 30 so in eine Aufbewahrungsposition am Bett 10 gebracht werden kann, ohne dass für den Patienten 90 nachteilig empfundene Störgrößen verbleiben.

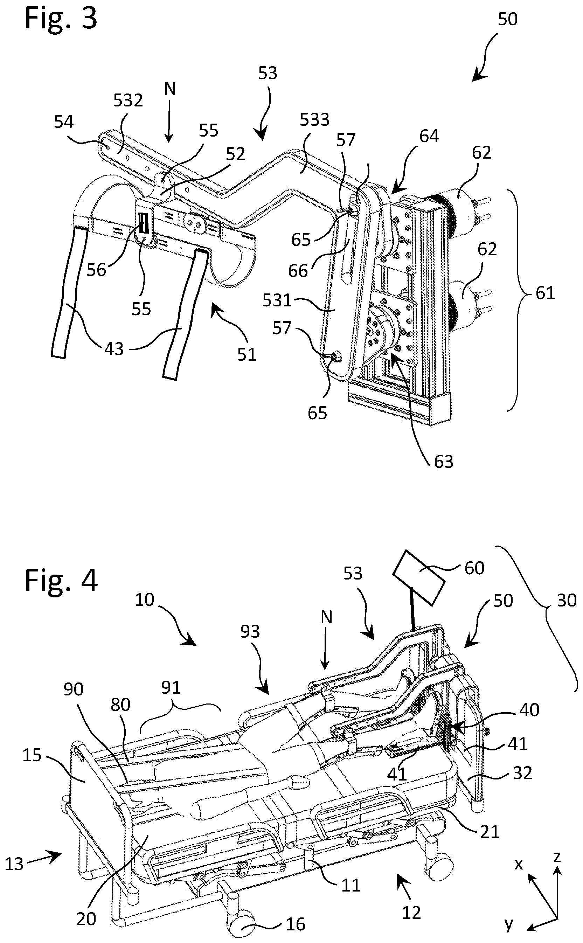

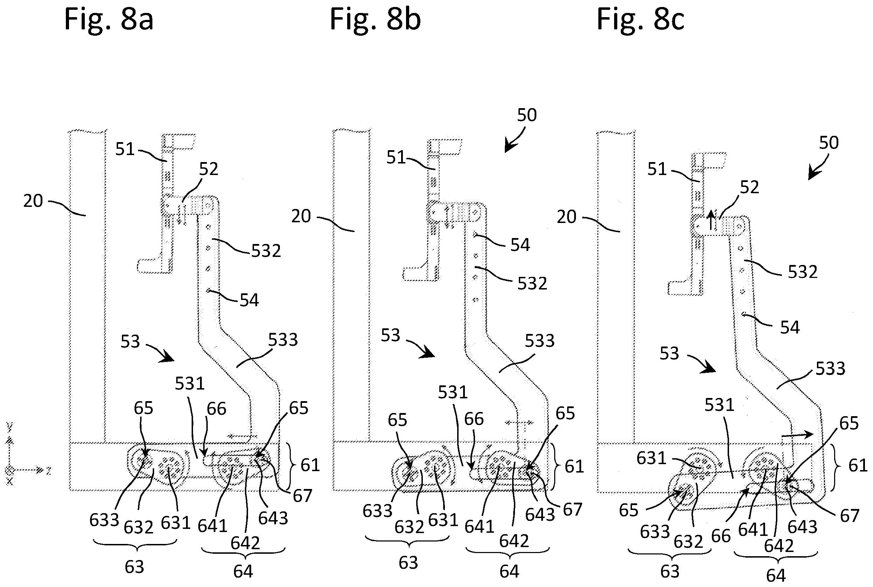

Fig. 3 zeigt in einem Ausschnitt den linken Ausleger 53 des Kniemoduls 50 des in Fig. 2 gezeigten Rehabilitationsmechanismus 30 in einer perspektivischen Ansicht. Erkennbar ist, wie das distale Ende bzw. Abschnitt 531 und das proximale Ende bzw. Abschnitt 532 des Auslegers 53 über ein Zwischenabschnitt 533 miteinander verbunden sind, welcher vorzugsweise so überbrückend ausgebildet ist, dass ein hinreichender Freiraum für den Fuß 94 des Patienten 90 sichergestellt ist. Darüber hinaus kann der über das Verbindungselement 52 eine Knie-Orthese 51 tragende proximale Abschnitt 532 des Ausleger 53 mittels axialer Sicherelemente wie beispielsweise Klemmen, Hebel, Klickverbinder (nicht dargestellt) oder dargestellt mittels zweier beispielsweise auf die Steuerbolzen 633 und 643 (siehe dazu Fig. 8 bis 11) aufschraubbarer Flügelmuttern 57 mit zwei motorgetriebenen Exzentern 63 und 64 verbunden sein.

Bevor die Therapie gestartet oder eine Vertikalisierung des Bettes 10 durchgeführt wird, bringt der Therapeut das distale Ende des Kniemoduls 50, d. h. das distale Ende 531 eines Auslegers 53 über die lösbare Bolzenverbindung (beispielsweise Flügelmutter 57 i.V.m. Steuerbolzen 633 bzw. 643) an der Tragplatte 32 und insbesondere an den für den Antrieb (Motor 45) des Auslegers 53 verwendeten Exzentern 63, 64 an. Am proximalen Ende 532 des Auslagers 53 des Kniemoduls 50 wird die Knie-Orthese 51 dem Patienten 90 am Knie 93 angelegt und von dem Therapeuten über Fixierbänder 43 an Unter- und Oberschenkel des Patienten 90 befestigt.

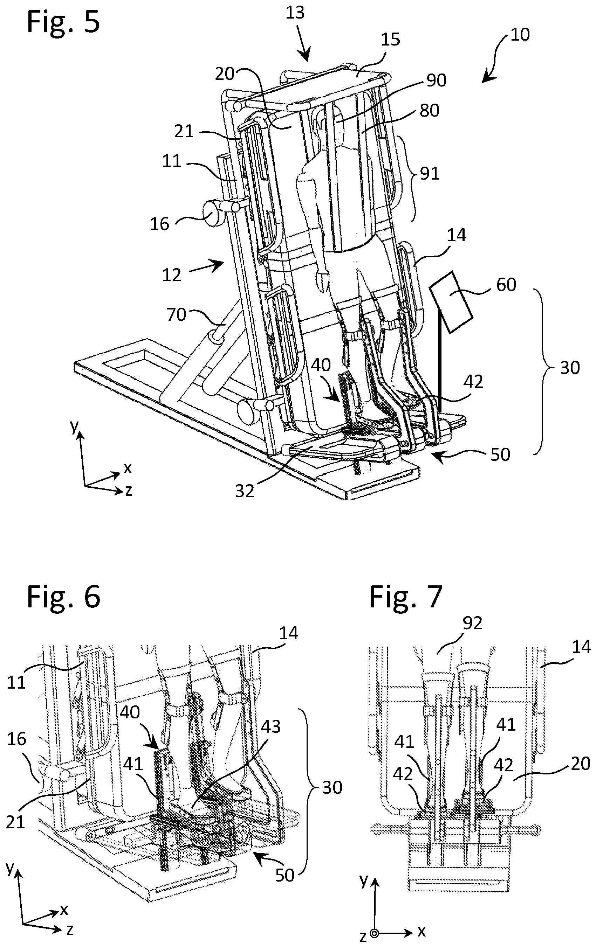

Soweit zusätzlich zum Kniemodul 50 ein Fußmodul 40 bevorzugt ist, lässt sich dieses vorzugsweise über einen Linearmechanismus 41 in y-Richtung, zur Anpassung an die Körperlänge des Patienten 90, und in x-Richtung, zur Anpassung an die Breite der Beine 90 verschieben und anschließend, beispielsweise über einen Klemmmechanismus (nicht dargestellt), an der Tragplatte 32 fixieren. Patientenseitig wird der Fuß des Patienten 90 über Fixierbänder 43 an der Trittfläche 42 des Fußmoduls 40 befestigt (Fig. 6)

Die Tragplatte 32 besitzt somit einen Linearmechanismus 41, um ein Fußmodul 40 innerhalb der Tragplatte 32 bewegen zu können. Weiterhin sind bevorzugt mindestens die Elektromotoren 62 zum Antrieb der je zwei Exzenter 63, 64 pro Ausleger 53, das Steuermodul 60, sowie die Exzenter 63, 64 selbst in die Tragplatte 32 integriert. Zur Übertragung der Lasten auf das Bett 10, muss die Verbindung zwischen Tragplatte 32 und Bett 10 eine hohe Steifigkeit aufweisen. Für den Einsatz des Rehabilitationsmechanismus 30 in handelsüblichen Betten 10 ist somit eine statisch modifizierte Tragplatte 32 bevorzugt.

**Fig. 4** zeigt das Bett 10 aus Fig. 2 mit einem am (Bett 10 gesicherten) Patienten 90 und mit angelegten Knie- 50 und Fußmodul 40 eines Rehabilitationsmechanismus 30 vor einer Vertikalisierung des Bettes 10 in einer perspektivischen Seitenansicht.

**Fig. 5** zeigt das Bett 10 aus Fig. 4 in einem vertikalisierten Zustand, **Fig. 6** den Rehabilitationsmechanismus 30 aus Fig. 5 in einer vergrößerten perspektivischen Ansicht und **Fig. 7** den Rehabilitationsmechanismus 30 zur Erzeugung einer Schrittbewegung etc. aus Fig. 6 in einer Vorderansicht.

Der Rehabilitationsmechanismus 30, erfindungsgemäß bestehend aus Steuermodul 60 und dem Kniemodul 50 mit Knie-Orthese 51, Verbindungselement 52 und Ausleger 53, sowie unter anderem bevorzugt mit dem Fußmodul 40 mit Trittfläche 42 und Fixierbändern 43, sowie dem Bett 10, wird für die Anwendung und die Dauer der Therapie über den Verstellmechanismus 70 in eine vertikale Position gebracht. Der Patient 90 wird, zusätzlich zu Fuß- 40 und Kniemodul 50, durch einen Stabilisierungsmechanismus 80 im Bett gestützt.

Der Rehabilitationsmechanismus 30 zur Erzeugung planmäßiger Rehabilitationsbewegungen wie insbesondere einer Schrittbewegung und/oder einer Treppensteigen-simulierenden Bewegung der Beine 92 des Patienten 90, sowie zur automatisierten Adjustierung nach einer Veränderung der Kontaktpunkte zwischen der die Gliedmaßen des Patienten aufnehmenden Knie-Orthese 51 (Kniegelenk 93 soll dabei stets auf Höhe eines zentralen Gelenkes der Orthese 51 liegen) und dem Rehabilitationsmechanismus 30, wird im Folgenden beschrieben.

Die Ausleger 53, welcher über das Verbindungselement 52 und die Knie-Orthese 51 eine Bewegung der Beine 92 des Patienten 90 induzieren, werden jeweils durch eine Rotation zweier Exzenter 63, 64 angetrieben. Die Rotation der Exzenter 63, 64 wird wiederum über den Antrieb der Welle 631 des ersten bzw. der Welle 641 des zweiten Exzenters 631, 641 durch einen Elektromotor 62 erzeugt. Die Bewegung wird über die Steuerbolzen 633 bzw. 643 des ersten und des zweiten Exzenters 632, 642 auf den jeweiligen Ausleger 53 übertragen. Der jeweilige Ausleger 53 ist auf die Steuerbolzen 632, 642 aufgesetzt und gegen axiale Verschiebung gesichert. Der Steuerbolzen 633 des ersten Exzenters 632 besitzt aufgrund eines rotatorischen Gleitlagers 65, einen rotatorischen Freiheitsgrad relativ zum Ausleger 53. Der Steuerbolzen 643 des zweiten Exzenters 642 besitzt aufgrund einer Linearführung 66, hier als Gleitstein 67 gezeichnet (siehe Fig. 3), und eines rotatorischen Gleitlagers innerhalb der Linearführung 66, sowohl einen translatorischen, als auch einen rotatorischen Freiheitsgrad in Relation zu dem Ausleger 53.

Das Verbindungselement 52 kann zur Anpassung des Rehabilitationsmechanismus' 30 an die unterschiedlichen Anthropometrien der Patienten 90 und an verschiedene Abstände des Patienten 90 zur Tragplatte 32 in unterschiedlichen Aufnahmepunkten 54 oder mittels eines Linearschlittens (nicht dargestellt) am Ausleger 53 angebracht werden. Das Verbindungselement 52 ist (mindestens einseitig, zur Adjustierung ggf. zunächst auch beidseitig) gelenkig gelagert (und später zumindest einseitig festsetzbar) an der Knie-Orthese 51 befestigt. Die Verbindung des Verbindungselements 52 zum Ausleger 53 besitzt bis einschließlich zur Einstellung der endgültigen Position des Patienten 90 einen rotatorischen Freiheitsgrad. Mit dem Beginn der Therapie wird dieser Freiheitsgrad bevorzugt, z.B. durch manuelles Einrasten, entfernt, sodass die Verbindung zwischen Verbindungselement 52 und Ausleger 53 während einer Therapie starr ist.

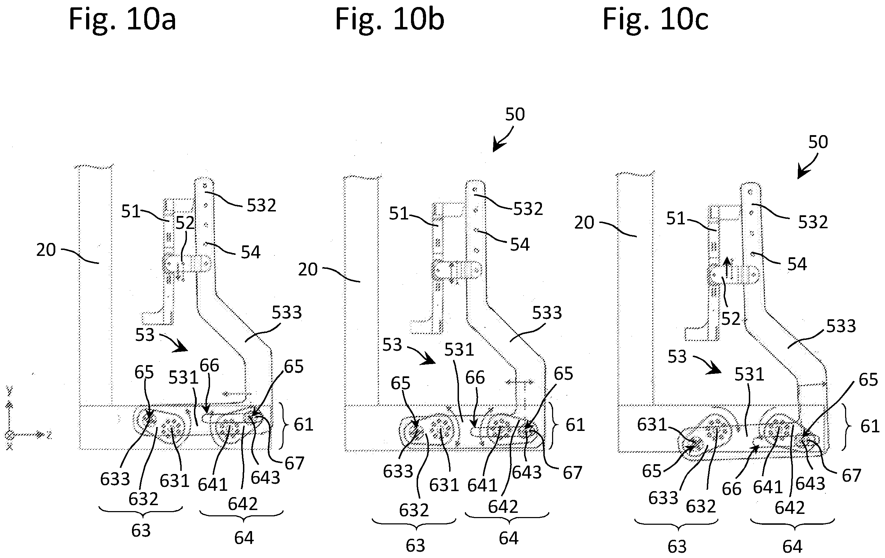

Fig. 8a bis c bzw. 10a bis 10c zeigen eine automatisierte Adjustierung nach Veränderung der Kontaktpunkte zwischen dem Rehabilitationsmechanismus 30 und den Gliedmaßen eines Patienten 90 mit langen (**Fig. 8a bis c**) bzw. mit kurzen Beinen 92 (**Fig. 10a bis c**).

Wie man den Fig. 8a bis c entnehmen kann weist das Verbindungselement 52 in Aufnahmepunkt 54 am Ausleger 53 einen größten Abstand zum distalen Ende bzw. Abschnitt 531 des Auslegers 53 auf als bei einem Patienten 90 mit geringer Beinlänge. Dieser Fall ist in den Fig. 10a bis c dargestellt, welchen man entnehmen kann, dass das Verbindungselement 52 in Aufnahmepunkt 54 am Ausleger 53 einen geringsten Abstand zum distalen Ende bzw. Abschnitt 531 des Auslegers 53 aufweist.

Figur 8a bzw. 10a zeigen den Ausgangszustand nach manueller Befestigung von Knie-Orthese 51 und Verbindungselement 52 am Ausleger 53. Durch eine Vertikalisierung des Bettes 10 rutscht der Patient 90 und somit dessen Gelenkpunkte in der Regel in negative y-Richtung. Durch die Kombination einer definierten rotatorischen Bewegung am Steuerbolzen 632 des ersten Exzenters 63 (Rotation der Welle 631 des ersten Exzenters 63 um x-Achse) mit einer definierten rotatorischen und translatorischen Bewegung am Steuerbolzen 642 des zweiten Exzenters 64 (Rotation der Welle 641 des zweiten Exzenters 641 um x-Achse, translatorische Bewegung des Gleitsteins 67 entlang des Auslegers 53) kann eine translatorische Bewegung des Verbindungselements 52 entlang der y-Achse ebenfalls in negativer Richtung erzielt werden, sodass die durch das Verrutschen des Patienten 90 verursachte Verschiebung der Gelenkpunkte, hier in negative y-Richtung, kompensiert werden kann. Im Allgemeinen können, wie in Fig. 8b und Fig. 10b dargestellt, Bewegungen des Verbindungselements 52 in positive und negative y-Richtung durch den Mechanismus ausgeführt werden. Die Figuren 8c bzw. 10c zeigen die Ausrichtung der Exzenter 63, 64 nach automatisierter Adjustierung an ein Verrutschen des Patienten 90 in negative y-Richtung.

Ziel der automatisierten Adjustierung ist die Krafteinleitung N in einem definierten, meist, aber nicht ausschließlich rechtem Winkel, auf die Knie-Orthese 51. Durch Verwendung zumindest eines Winkelsensors 55 in den Gelenken des Verbindungselements 52, sowie alternativ oder kumulativ eines Kraftsensors 56 zwischen Verbindungselement 52 und Knie-Orthese 51 kann eine Fehlstellung der Gelenkpunkte nach Vertikalisierung, oder anderweitigem Verrutschen des Patienten 90 detektiert werden, sodass der Vorgang der automatisierten Adjustierung ausgelöst und, wie oben beschrieben, ausgeführt wird. Dies hat einerseits eine automatische Nachjustierung bei Abweichung "idealer" Winkel & Kräfte zum Vorteil. Ideale Winkel könnten z.B. so definiert sein, dass eine Kraft N immer senkrecht zur Matratze 20 eingebracht wird, oder tangential zur Kreisbewegung (bzw. den Trajektorien T), welche das Knie 93 um den Hüftdrehpunkt beschreibt. Die Definition von "ideal" wird von Therapeuten zu definieren sein und kann ggf. von Patient 90 zu Patient 90 unterschiedlich ausfallen. Die vorliegende Erfindung eröffnet somit dem Therapeuten später eine Wahl, wie die Kräfte N eingebracht werden, je nachdem was der Patient 90 benötigt, und dies müsste nicht von vorne herein vorgeschrieben sein sondern könnte zur Laufzeit geändert werden, so dass das Rehabilitationsmechanismus 30 noch besser an jeden Patienten 90 individuell angepasst werden kann.

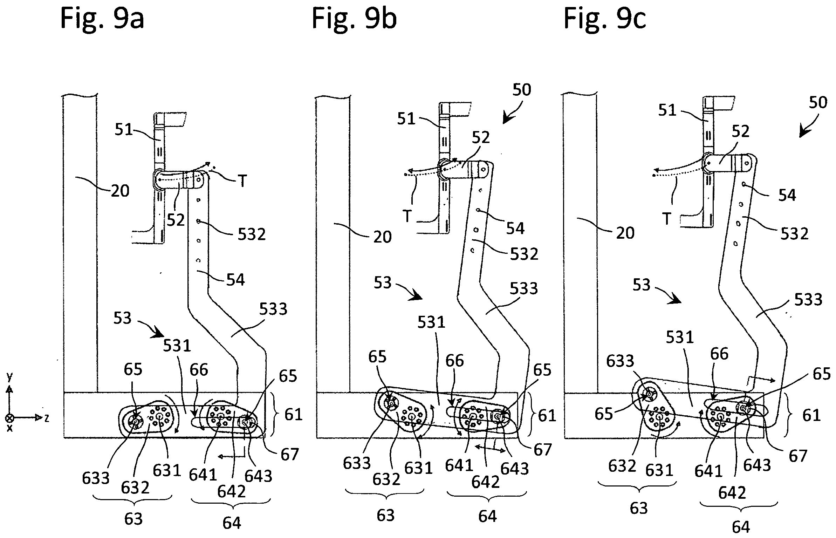

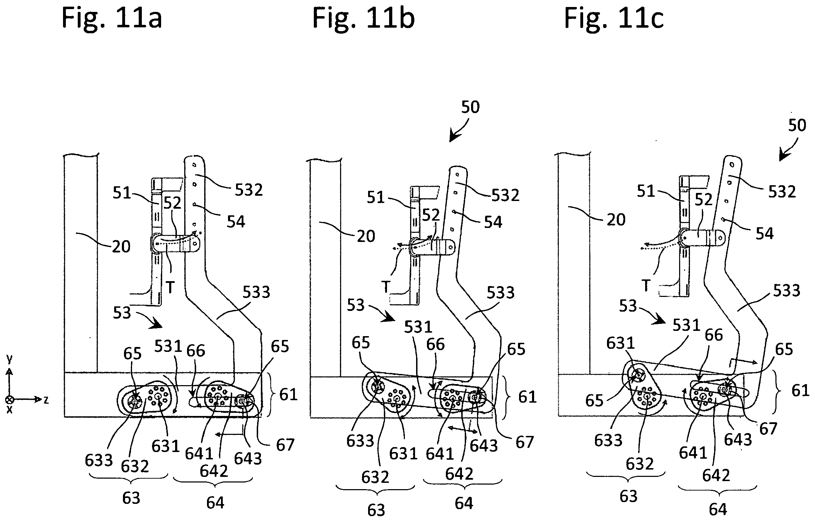

Fig. 9a bis c bzw. 11a bis c zeigen die von dem Rehabilitationsmechanismus 30 über die Knie-Orthese 51 erzeugten Trajektorien (T) zum Beugen bzw. Strecken der Beine 92 eines Patienten 90 mit langen (**Fig. 9a bis c**) bzw. mit kurzen Beinen 92 (**Fig. 11a bis c**).

Wie man den Fig. 9a bis c entnehmen kann weist das Verbindungselement 52 in Aufnahmepunkt 54 am Ausleger 53 einen größten Abstand zum distalen Ende bzw. Abschnitt 531 des Auslegers 53 aus als bei einem Patienten 90 mit geringer Beinlänge. Dieser Fall ist in den Fig. 11a bis 11c dargestellt, welchen man entnehmen kann, dass das Verbindungselement 52 in Aufnahmepunkt 54 am Ausleger 53 einen geringstem Abstand zum distalen Ende bzw. Abschnitt 531 des Auslegers 53 aufweist. In beiden Fällen kann bei einem jeden Patienten 90, gleich ob mit langen oder kurzen Beinen 92, eine Schrittbewegung und/oder einer Treppensteigen-simulierende Bewegung erzeugt werden.

Dabei ist in den Figuren 9a bzw. 11a der Ausgangszustand, in welchem sich Verbindungselement 52 und somit die Knie-Orthese 51 am niedrigsten Punkt der z-Achse befinden, dargestellt, in welchem das Bein 92 des Patienten 90 gestreckt ist.

Durch eine Kombination definierter Rotationen der Exzenter 63, 64 können die in den Figuren 9b bzw. 11b dargestellten, beispielhaften Trajektorien (T) abgefahren werden, welche annäherungsweise einer Rotation des Kniegelenks 93 um das Hüftgelenk des Patienten 90 entsprechen. Für eine Bewegung des Verbindungselements 52 in positive y- und z-Richtung rotiert der erste Exzenter 63 in positive, der zweite Exzenter 64 in negativer Richtung um die x-Achse. Die Linearführung im Gleitlager 66 führt eine Bewegung in der y-z-Ebene in Richtung des Drehpunkts des ersten Exzenters 63 aus, dargestellt in den Figuren 9a bzw. 11a.

Für die Bewegung in jeweils negative y- und z-Richtung, dargestellt in den Figuren 9c/11c, verhalten sich die Rotationen der Exzenter 63, 64, sowie die translatorische Bewegung der Linearführung im Gleitlager 66, entgegengesetzt. Die Figuren 9c bzw. 11 c zeigen weiterhin die Ausrichtung der Exzenter 63, 64 bei maximaler Auslenkung des Verbindungselements 52 in z-Richtung und somit die Position der maximalen Beugung eines Beines 92 des Patienten 90.

Zwischen den Therapie-Einheiten können sowohl Knie- 50 als auch ein etwaiges Fußmodul 40 durch den Therapeuten vom Patienten 90 abgenommen und derart am Bett 10 oder alternativ im Krankenzimmer aufbewahrt werden, dass einerseits ausreichend Bewegungsfreiraum für den Patienten 90 zwischen den Therapie-Einheiten gewährleistet und andererseits dem Therapeuten eine schnelle Wiederanbringung der Komponenten an Patient 90 und Bett 10 bzw. Tragplatte 32 ermöglicht wird. Das Fußmodul 40 kann hierfür über den zur Anpassung an die Beinbreite verwendeten Linearmechanismus in x-Richtung an den äußeren Rand der Matratze 20 verschoben und arretiert werden. Die Ausleger 53 können durch ein Lösen der beispielsweise mittels Flügelmuttern 57 gesicherten Bolzenverbindung 633 und 634 von den Exzentern 63, 64 abgenommen und zur Seite abgeklappt werden. Die Knie-Orthese 51 mit Verbindungselement kann am Rücken der Fußplatte 32 befestigt werden (vgl. Fig. 2).

Die vorliegende Erfindung stellt einen gegenüber dem Stand der Technik verbesserten Rehabilitationsmechanismus 30 bereit, welcher sich problemlos in alle bekannten Klinikabläufe integrieren lässt, und zwar sowohl für insbesondere aufgrund orthopädischer, intensivmedizinischer und/oder neurologischer Aktivitätseinschränkungen bettpflichtig gewordener Patienten 90. Ohne diese Patienten 90 umbetten zu müssen gestattet die vorliegende Erfindung eine planmäßig automatisierte Rehabilitation zumindest der Gelenke, Muskeln und Sehnen der Beine 92 bettpflichtiger Patienten 90. Aufgrund seines modularen Aufbaus kann der Rehabilitationsmechanismus 30 schnell entfernt werden und ist weder im Notfall noch im Klinik Alltag hinderlich. Die Möglichkeit einer Belastung der Füße 94 ganz oder teilweise mit der Gewichtskraft des Patienten 90 trainiert weiterhin die Muskulatur und das Skelett und verhindert eine Degeneration des muskulo-skeletalen Systems. Eine Möglichkeit zur Vertikalisierung trainiert zudem das kardiovaskuläre System.

Dies ist bei orthopädischen ebenso wie bei intensivpflichtigen oder neurologischen Patienten 90 gleich wichtig. Neben handelsüblichen oder eigens geschaffenen Pflege- oder Krankenbetten 10 ist ein Rehabilitationsmechanismus 30 nach der Erfindung leicht insbesondere auch in handelsüblichen oder eigens geschaffenen Spital- oder Intensivbetten 10 anbringbar und entfernbar, und zwar unabhängig davon, ob der bettpflichtige Patient 90 in dem jeweiligen Bett 10 ganz oder teilweise in eine vertikalisierte Lage gebracht werden kann, wobei in einer jeden vom bettpflichtigen Patienten 10 zwischen einer horizontal und einer vertikalisiert eingenommenen Lage der Rehabilitationsmechanismus 30 eine rhythmische Be- und Entlastung der Fußsohlen 95 bettpflichtiger Patienten 90 unterstützt.

Insbesondere kann mit der vorliegenden Erfindung erstmals ein Verfahren zur Ansteuerung eines Rehabilitationsmechanismus 30 realisiert werden, bei dem

- mittels wenigstens eines Winkelsensors 55 der vom Verbindungselement 52 zur Knie-Orthese 51 und/oder zum Ausleger 53 eingenommene Winkel ($\varphi$);

und/oder

- mittels eines Kraftsensor 56 die über den Ausleger 53 und das Verbindungselement 52 in die Knie-Orthese 51 eingeleitete Kraft (N)

überwacht werden, wobei bei Über- oder Unterschreitung einer variabel vorgegebenen Soll-Winkelschwelle ($\varphi\_soll$) und/oder bei Über- oder Unterschreitung einer variabel vorgegebenen Soll-Kraftschwelle das Steuermodul 60 die Mechanik 61 dergestalt ansteuert, dass der Ist-Winkel ($\varphi\_ist$) und/oder die Ist-Kraft (N) durch Adjustierung der Exzenter 63 und 64 bzw. deren Exzenterscheiben 632, 642 unterhalb der überschrittenen oder oberhalb der unterschrittenen Schwellen auf die Soll-Winkelschwelle ($\varphi\_soll$) und/oder die Soll-Kraftschwelle zurückgeführt werden.

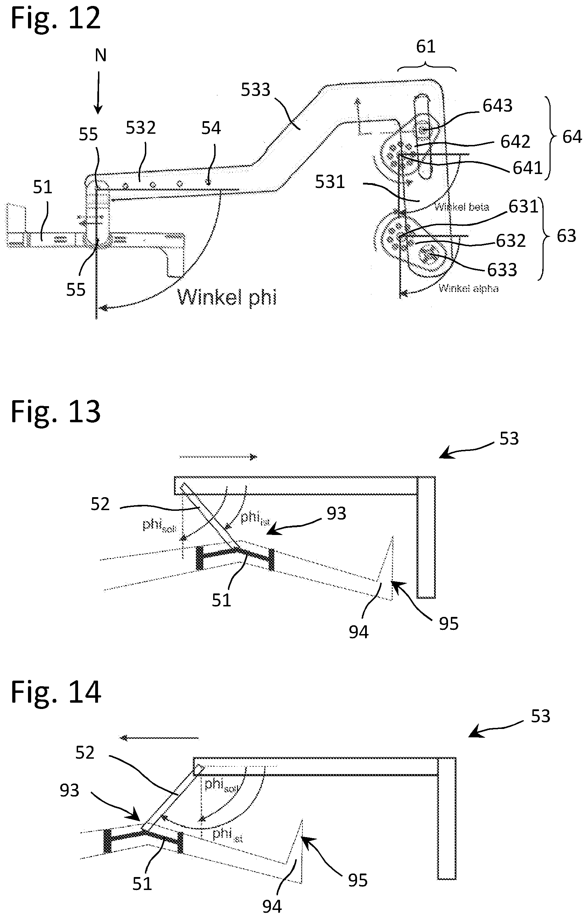

Fig. 12 zeigt beispielhaft anhand des Auslegers 53 aus Fig. 8c in einer vergrößerten Darstellung, wie mittels wenigstens eines Winkelsensors 55 der vom Verbindungselement 52 zur Knie-Orthese 51 und/oder zum Ausleger 53 eingenommene Winkel phi ($\varphi$) überwacht werden kann. Weiterhin dargestellt sind die Winkel alpha ($\alpha$) und beta ($\beta$), welche jeweils ebenfalls durch einen Winkelsensor (nicht dargestellt) messbar sind oder eine definierte Dreh-Winkel-Stellung des den ersten 63 bzw. den zweiten 64 Exzenter antreibenden Motors 62 repräsentieren.

Der durch wenigstens einen Winkelsensor 55 überwachte Winkel $\varphi$ zwischen Ausleger 53 und Verbindungselement 52, welches den Ausleger 53 mit der Knie-Orthese 51 verbindet, soll eine jede therapeutisch erwünschte Winkelschwelle ($\varphi\_soll$) einnehmen können. Eine wünschenswerte Winkelschwelle ($\varphi\_soll$) kann beispielsweise mit 90° zur Matratze 20 angegeben sein. Bei diesen 90° ist weiterhin bevorzugt, dass der Kontaktpunkt, an dem der Ausleger 53 mit dem Verbindungselement 52 verbunden ist, genau oberhalb des Drehpunktes der Knie-Orthese 51 liegt.

Fig. 13 zeigt, wie abweichend von einer Sollwinkelstellung ($\varphi\_soll$) das Kniegelenk 93 eines Patienten 90 in Richtung des Fußendes des Bettes 10 verrutscht sein kann. In diesem Fall I muss der Ausleger 53 auf einen neuen, korrekten Kontaktpunkt nachjustiert werden, da sonst die Kraft (N), welche über den Ausleger 53, das Verbindungselement 52 und die Knieorthese 51 auf das Kniegelenk 93 eingebracht werden soll, insbesondere nicht senkrecht eingebracht werden kann.

In dem in Fig. 13 gezeigten Fall I müssen die Exzenter 63 und/oder 64 adjustiert werden, d.h. wie in Fig. 8a gezeigt, gegeneinander verdreht werden (und zwar der zum Winkel alpha gehörende Exzenter 63 entgegen den Uhrzeigersinn, also in negative Drehrichtung und der zum Winkel beta gehörende Exzenter 64 im Uhrzeigersinn, also in positive Drehrichtung), um den Ausleger 53 wie in Fig. 13 mittels eines Pfeils gezeigt linear in Richtung des Fußendes des Bettes 10 zu verschieben. Dies kann beispielsweise durch eine Regelung erfolgen, welche den aktuellen Ist-Winkel phi ($\varphi\_ist$) misst und diesen auf eine variabel vorgegebene Soll-Winkelschwelle ($\varphi\_soll$) von z.B. bevorzugt 90 Grad einregelt, beispielsweise mittels eines sog. PID Reglers (proportional-integral-derivative controller).

Fig. 14 zeigt, wie abweichend von einer Soll-Winkelstellung ($\varphi\_soll$) das Kniegelenk 93 eines Patienten 90 in Richtung des Kopfendes des Bettes 10 verrutscht sein kann. Auch in diesem Fall II muss der Ausleger 53 auf einen neuen, korrekten Kontaktpunkt nachjustiert werden, da sonst wiederum eine Kraft (N), welche über den Ausleger 53, das Verbindungselement 52 und die Knie-Orthese 51 auf das Kniegelenk 93 eingebracht werden soll, insbesondere nicht senkrecht eingebracht werden kann.

In dem in Fig. 14 gezeigten Fall II müssen die Exzenter 63 und/oder 64 ebenfalls adjustiert werden, d.h. wie in Fig. 8c gezeigt, gegeneinander verdreht werden (und zwar der zum Winkel alpha gehörende Exzenter 63 im Uhrzeigersinn, also in positive Drehrichtung und der zum Winkel beta gehörende Exzenter 64 entgegen den Uhrzeigersinn, also in negative Drehrichtung), um den Ausleger 53 wie in Abbildung 14 mittels eines Pfeils gezeigt, linear in Richtung des Kopfendes des Bettes 10 zu verschieben. Dies kann beispielsweise durch eine Regelung erfolgen, welche den aktueSoll-llen Ist-Winkel phi (φ ist) misst und diesen auf eine variabel vorgegebene Winkelschwelle (φ_soll) von z.B. bevorzugt 90 Grad einregelt insbesondere mittels eines PID Reglers.

Die zuvor beschriebene Adjustierung der Exzenterscheiben 632, 642 kann erfindungsgemäß bevorzugt sequentiell (also unabhängig von planmäßig durchgeführten Rehabilitationsbewegungen) oder zeitgleich zu planmäßig durchgeführten Rehabilitationsbewegungen erfolgen.

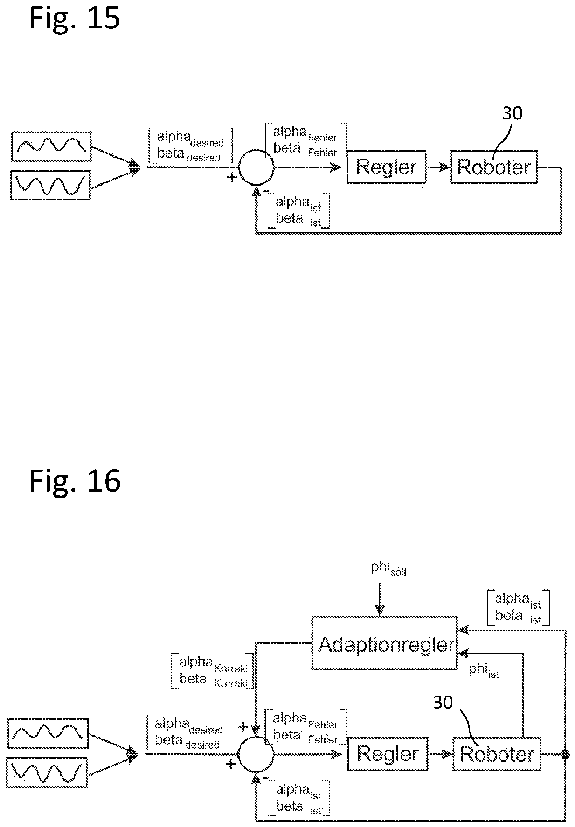

**Fig. 15** zeigt anhand einer Prinzipskizze einen Regelkreis zur Erzeugung einer planmäßigen Rehabilitationsbewegung mittels eines Rehabilitationsmechanismus 30, in Fig. 15 auch als "Roboter" bezeichnet. Demgemäß wird in einem ersten Schritt die Exzenterwinkel alpha(t) und beta(t) als zeitliche Abfolge von gewünschten Winkelkombinationensparen $\begin{bmatrix} alpha(t) \\ beta(t) \end{bmatrix}$ festgelegt. Der Zusammenhang zwischen der Änderung der Winkel alpha und beta und der daraus resultierenden Bewegung des Auslegers 53 ist in den Figuren 9a bis c dargestellt und beschrieben. Durch geeignete Ansteuerung wird das Kniegelenk 93 eines Patienten 90 vorzeilhaft so bewegt, dass das Kniegelenk 93 sich auf einer kreisförmigen Trajektorie T um das Hüftgelenk bewegt.

Durch Abgleich des Ist-Winkels (φ_ist) mit dem Soll-Winkel (φ_soll) kann ein alpha/beta Fehler berechnet werden. Dieser kann dann von einem Regler, z.B. einem PID Regler, ausgeregelt werden.

**Fig. 16** schließlich zeigt anhand einer Prinzipskizze einen gegenüber Fig. 15 erweiterten Regelkreis zur Erzeugung einer sequentiellen oder zeitgleich zu planmäßigen Rehabilitationsbewegungen erfolgenden Adjustierung der Exzenter 63 und/oder 64 eines Rehabilitationsmechanismus 30, auch in Fig. 16 exemplarisch als "Roboter" bezeichnet.

Wird nun eine planmäßige Rehabilitationsbewegung durchgeführt, so kann es sein dass der Patient 90 aufgrund der Krafteinwirkung N und/oder aufgrund von Schwerkraft verrutscht. Dies kann z.B. insbesondere auch passieren, wenn das Bett 10 vertikalisiert wird und die Gravitations- bzw. Schwerkräfte den Patienten 90 nach unten drücken.

Mit den in Fig. 16 dargestellten Regelkreis kann nun gleichzeitig zur planmäßigen Rehabilitationsbewegung die Adjustierung der Exzenter 63 und/oder 64, also die Exzenterbewegung alpha und beta durchgeführt werden. Dabei kann insbesondere zusätzlich ein Korrekturterm eingefügt werden, welcher eine Anpassung insbesondere der Position des Proximalen Abschnitts 532 des Auslegers 53 durchführt, so dass der Ist-Winkel phi (φ_ist) zu jeder Zeit seinem gewünschten Soll-Wert $phi_{soll}$ (φ_soll) folgt. Dies bedeutet - mathematisch beschrieben - innerhalb des Regelsystems eine Veränderung des Phasenverzuges zwischen den Exzenterrotationskommandos alpha(t) und beta(t).

Somit können planmäßige Rehabilitationsbewegung und Anpassungen der Position des/der Kontaktpunktes gleichzeitig durchgeführt werden.

Alternativ hierzu kann natürlich auch sequenziell, d.h. nacheinander, gearbeitet werden, also zuerst für eine bestimmte Zeit die planmäßige Rehabilitationsbewegung durchgeführt, und dann eine erneute Adjustierung durchzuführen werden.

Die vorliegende Erfindung hat des Weiteren zum Vorteil, dass von einem Therapeut voreingestellt werden kann, welchen $phi_{soll}$-Wert das Steuermodul 60 ausregelt.

- Eine Möglichkeit wäre es, Phi (φ_soll) immer auf 90 Grad zu halten.
- Eine weitere Möglichkeit wäre es, die Position des Auslegers 53 über die Winkel alpha und beta zu berechnen und darüber den Winkel $phi_{soll}$ (φ_soll) in Abhängigkeit von alpha und beta immer so zu bestimmen, dass das Verbindungselement 52 zwischen Ausleger 53 und Orthese 51 stets senkrecht auf der Matratze 20 steht.
- Eine dritte Möglichkeit wäre, den Winkel φ_soll so zu regeln, dass das Verbindungselement 52 zwischen Ausleger 53 und Knie-Orthese 51 immer tangential zu der Kreisbahn, d.h. zu der kreisförmigen Trajektorie T um das Hüftgelenk, ausgerichtet ist, welche das Kniegelenk 93 um den Hüftdrehpunkt ausführt. Damit wäre φ_soll zeitabhängig von der vorgegebenen Winkelkombinationensparen $\begin{bmatrix} alpha(t) \\ beta(t) \end{bmatrix}$ der Exzenter 63 und 64, und würde als φ_soll(t) bezeichnet werden.

Diese Entscheidungen kann schließlich ein Therapeut in einer bevorzugten Ausgestaltung vorteilhaft an einem Bedienteil des Steuermoduls 60 einstellen.

**Bezugszeichenliste**

| | |
|---|---|
| 10 | Bett, insbesondere: Pflegebett, Krankenbett, Spitalbett oder Intensivbett |
| 11 | Bettrahmen |
| 12 | Längsseiten |
| 13 | Querseiten |
| 14 | Längsseitenbegrenzung |
| 15 | Querseitenbegrenzung |
| 16 | Rollbeine |

20 Matratze
21 Matratzenrahmen

30 Rehabilitationsmechanismus
32 am Bett- 11 oder Matratzenrahmen 21 festsetzbare Tragplatte für Rehabilitationsmechanismus 30

40 Fußmodul
41 Feststellmittel
42 Trittfläche
43 Fixierbänder

50 Kniemodul
51 Knie-Orthese
52 Verbindungselement
53 Ausleger
531 Distaler Abschnitt des Auslagers 53
532 Proximaler Abschnitt des Auslegers 53
533 Zwischenabschnitt des Auslegers 53
54 Aufnahmepunkte
55 Winkelsensor
56 Kraftsensor
57 Flügelmutter
60 Steuermodul

61 Mechanik
62 Elektromotor

63 erster Exzenter
631 Exzenterwelle des ersten Exzenters 63
632 Exzenterscheibe des ersten Exzenters 63
633 Steuerbolzen des ersten Exzenters 63

64 zweiter Exzenter
641 Exzenterwelle des zweiten Exzenters 64
642 Exzenterscheibe des zweiten Exzenters 64
643 Steuerbolzen des zweiten Exzenters 64

65 Radiallager
66 Gleitlager
67 Gleitstein

70 Verstellmechanismus

80 Stabilisierungsmechanismus

90 Patient
91 Herz-/Lungen-Thorax
92 Bein
93 Kniegelenk
94 Fuß
95 Fußsohle

N Kraft
T Trajektorien

**Patentansprüche**

1. Rehabilitationsmechanismus (30) für ein Bett (10) mit sich in y-Richtung erstreckenden Längsseiten (12) sowie in x-Richtung erstreckenden Querseiten (13), geeignet ausgebildet für eine planmäßig automatisierte Rehabilitation zumindest der Gelenke, Muskeln und Sehnen der Beine (92) eines bettpflichtigen Patienten (90), wenigstens umfassend:

- ein in Wirkverbindung mit den Kniegelenken (93) des bettpflichtigen Patienten (90) bringbares Kniemodul (50),
- wenigstens einen Winkelsensor (55), welcher den von einem Verbindungselement (52) zu einer Knie-Orthese (51) und/oder zum Ausleger (53) eingenommenen Winkel überwacht; und
- ein Steuermodul (60) zur Erzielung planmäßiger Rehabilitationsbewegungen zumindest der Gelenke, Muskeln und Sehnen der Beine (92) des bettpflichtigen Patienten (90) mittels des Kniemoduls (50),

- wobei das Steuermodul (60) eingerichtet ist, bei Über- oder Unterschreitung einer variabel vorgegebenen, durch den Winkelsensor (55) überwachten, Soll-Winkelschwelle ($\varphi\_soll$) eine Mechanik (61) dergestalt anzusteuern, dass ein Ist-Winkel ($\varphi\_ist$) durch Adjustierung von Exzenterscheiben (632, 642) unterhalb der überschrittenen oder oberhalb der unterschrittenen Schwellen auf die Soll-Winkelschwelle ($\varphi\_soll$) zurückgeführt wird;

**dadurch gekennzeichnet, dass**

- das Kniemodul (50) wenigstens umfasst:

- eine in z-Richtung oberhalb einer Matratze (20) angeordnete Knie-Orthese (51) zur Aufnahme eines Kniegelenkes (93) des bettpflichtigen Patienten (90) bei Benutzung, wobei das Kniegelenk (93) dabei stets auf Höhe eines zentralen Gelenkes der Knie-Orthese (51) ist;
- ein mit der Knie-Orthese (51) verbundenes Verbindungselement (52);
- einen in z-Richtung oberhalb von Patient (90) und Matratze (20) angeordneten proximalen Abschnitt (532) eines Auslegers (53), an welchem das Verbindungselement (52) befestigt ist,
- und zur unmittelbaren oder mittelbaren Abstützung an einem Bett-(11) oder Matratzenrahmen (21) eine mittels des Steuermoduls (60) ansteuerbare Mechanik (61), welche über den proximalen Abschnitt (532) des Auslegers (53), das Verbindungselement (52) und

- einen Kontaktpunkt, an dem der proximale Abschnitt (532) des Auslegers (53) mit dem Verbindungselement (52) verbunden ist, und welcher genau oberhalb des Drehpunktes der Knie-Orthese (51) liegt,

eine definierte Kraft (N) dergestalt in z-Richtung genau oberhalb eines Drehpunkts der Knie-Orthese (51) in diese einleitet, dass über das darin bei Benutzung aufgenommene Kniegelenk (93) des bettpflichtigen Patienten (90) die Gelenke, Muskeln und Sehnen dieses Beines (92) planmäßige Rehabilitationsbewegungen ausführen, wobei

    - die Mechanik (61) zwei Exzenter (63, 64) umfasst, welche jeweils durch eine auf einer Exzenterwelle (631, 641) angebrachten Exzenterscheibe (632, 642), deren Mittelpunkt jeweils außerhalb der Wellenachse liegt, gebildet sind;
    - die Exzenterwellen (631, 641) durch einen Elektromotor (62) angetrieben sind; und
    - ein distaler Abschnitt (531) des Auslegers (53) mit einem Steuerbolzen (633) der ersten Exzenterscheibe (632) über ein Radiallager (65) und mit einem Steuerbolzen (643) der zweiten Exzenterscheibe (642) über ein Gleitlager (66) wirkverbunden ist,
    - und wobei das Steuermodul (60) eingerichtet ist, die Soll-Winkelschwelle ($\varphi$_soll) in Abhängigkeit von den Exzenterwinkeln alpha und beta immer so zu bestimmen, dass das Verbindungselement (52) zwischen Ausleger (53) und Orthese (51) stets senkrecht auf der Matratze (20) steht.

**2.** Rehabilitationsmechanismus (30) nach Anspruch 1, **dadurch gekennzeichnet, dass** das Gleitlager (66) durch ein rotatorisch mit dem Steuerbolzen (643) des zweiten Exzenters (64) verbundenen Gleitstein (67) gebildet ist.

**3.** Rehabilitationsmechanismus (30) nach Anspruch 1 oder 2, weiterhin umfassend

    - einen Kraftsensor (57), welcher die über den Ausleger (53) und das Verbindungselement (52) in die Knie-Orthese (51) eingeleitete Kraft (N) überwacht.

**4.** Rehabilitationsmechanismus (30) nach Anspruch 3, **dadurch gekennzeichnet, dass** das Steuermodul (60) eingerichtet ist, bei Über- oder Unterschreitung einer variabel vorgegebenen, durch den Kraftsensor (56) überwachten, Soll-Kraftschwelle die Mechanik (61) dergestalt anzusteuern, dass die Ist-Kraft (N) durch Adjustierung der Exzenterscheiben (632, 642) unterhalb der überschrittenen oder oberhalb der unterschrittenen Schwellen auf die Soll-Kraftschwelle zurückgeführt wird.

**5.** Rehabilitationsmechanismus (30) nach einem der vorherigen Ansprüche, **dadurch gekennzeichnet, dass** das Steuermodul (60) eingerichtet ist, die Mechanik (61) dergestalt anzusteuern, dass die Adjustierung der Exzenterscheiben (632, 642) sequentiell oder zeitgleich zu planmäßig durchgeführten Rehabilitationsbewegungen erfolgt.

**6.** Rehabilitationsmechanismus (30) nach einem der vorherigen Ansprüche, **dadurch gekennzeichnet, dass** der Ausleger (53) mehrere, vorzugsweise in einem regelmäßigen Abstand, voneinander beabstandete Aufnahmepunkte (54) zur gelenkigen Lagerung des Verbindungselements (52) aufweist.

**7.** Rehabilitationsmechanismus (30) nach einem der vorherigen Ansprüche, **dadurch gekennzeichnet, dass** der Ausleger (53) eine den Fuß (94) eines Beines (92) umgreifende Aussparung (55) aufweist.

**8.** Rehabilitationsmechanismus (30) nach einem der vorherigen Ansprüche, weiterhin umfassend

    - ein in Wirkverbindung mit den Füßen (94) und/oder den Fußsohlen (95) des bettpflichtigen Patienten (90) bringbares Fußmodul (40).

**9.** Rehabilitationsmechanismus (30) nach Anspruch 8, **dadurch gekennzeichnet, dass** das Fußmodul (40) so ausgebildet ist, dass bei einer Rehabilitationsbewegung des Patienten (90) ein Drehmoment auf das Sprunggelenk des Patienten (90) aufgebracht wird.

**10.** Bett (10) für bettpflichtige Patienten (90), wenigstens umfassend einen Rehabilitationsmechanismus (30) nach einem der vorherigen Ansprüche.

**Claims**

**1.** Rehabilitation mechanism (30) for a bed (10) having longitudinal sides (12) running in a y direction and transverse sides (13) running in an x direction, suitably designed for a planned, automated rehabilitation of at least the joints, muscles and tendons of the legs (92) of a bedridden patient (90), the rehabilitation mechanism comprising at least:

    - a knee module (50) which can be operatively connected to the knee joints (93) of the bedridden patient (90),

- at least one angle sensor (55) which monitors the angle adopted by a connection element (52) to a knee orthosis (51) and/or to the extension arm (53); and
- a control module (60) for achieving planned rehabilitation movements of at least the joints, muscles and tendons of the legs (92) of the bed-ridden patient (90) by means of the knee module (50),

- wherein, if a variably predefined desired angle threshold ($\varphi$_desired) monitored by the angle sensor (55) is exceeded or undershot, the control module (60) is configured to actuate a mechanical device (61) in such a way that an actual angle ($\varphi$_actual) is returned, by adjustment of eccentric discs (632, 642), to the desired angle threshold ($\varphi$_desired) below the exceeded thresholds or above the undershot thresholds;

**characterized in that**

- the knee module (50) comprises at least:

- a knee orthosis (51) which is arranged in the z direction above a mattress (20) and serves to receive a knee joint (93) of the bed-ridden patient (90) during use, wherein the knee joint (93) is then always at the height of a central joint of the knee orthosis (51);
- a connection element (52) connected to the knee orthosis (51);
- arranged in the z direction above patient (90) and mattress (20), a proximal portion (532) of an extension arm (53) to which the connection element (52) is secured,
- and, to be supported directly or indirectly on a bed frame (11) or mattress frame (21), a mechanical device (61) which can be actuated by means of the control module (60) and which, via the proximal portion (532) of the extension arm (53), the connection element (52) and
- a contact point, at which the proximal portion (532) of the extension arm (53) is connected to the connection element (52) and which lies exactly above the rotation point of the knee orthosis (51),

introduces a defined force (N) into the knee orthosis (51), in the z direction exactly above a rotation point of said knee orthosis (51), in such a way that the joints, muscles and tendons of the leg (92) perform planned rehabilitation movements via the bedridden patient's knee joint (93) that is received in said knee orthosis during use, wherein

- the mechanical device (61) comprises two eccentrics (63, 64) which are each formed by an eccentric disc (632, 642) which is mounted on an eccentric shaft (631, 641) and whose centre point lies in each case outside the shaft axis;
- the eccentric shafts (631, 641) are driven by an electric motor (62); and
- a distal portion (531) of the extension arm (53) is operatively connected to a control pin (633) of the first eccentric disc (632) via a radial bearing (65) and is operatively connected to a control pin (643) of the second eccentric disc (642) via a sliding bearing (66),
- and wherein the control module (60) is configured to always determine the desired angle threshold ($\varphi$_desired) as a function of the eccentric angles alpha and beta, such that the connection element (52) between extension arm (53) and orthosis (51) is always perpendicular to the mattress (20).

2. Rehabilitation mechanism (30) according to Claim 1, **characterized in that** the sliding bearing (66) is formed by a slide block (67) connected rotatably to the control pin (643) of the second eccentric (64).

3. Rehabilitation mechanism (30) according to Claim 1 or 2, further comprising

- a force sensor (57) which monitors the force (N) introduced into the knee orthosis (51) via the extension arm (53) and the connection element (52).

4. Rehabilitation mechanism (30) according to Claim 3, **characterized in that**, if a variably predefined desired force threshold monitored by the force sensor (56) is exceeded or undershot, the control module (60) is configured to actuate the mechanical device (61) in such a way that the actual force (N) is returned, by adjustment of the eccentric discs (632, 642), to the desired force threshold below the exceeded thresholds or above the undershot thresholds.

5. Rehabilitation mechanism (30) according to one of the preceding claims, **characterized in that** the control module (60) is configured to actuate the mechanical device (61) in such a way that the adjustment of the eccentric discs (632, 642) is effected sequentially or simultaneously with respect to planned rehabilitation movements that are performed.

6. Rehabilitation mechanism (30) according to one of the preceding claims, **characterized in that** the extension arm (53) has a plurality of receiving points (54) for mounting the connection element (52) in an articulated manner, said receiving points (54) being spaced apart from one another, preferably at regular intervals.

7. Rehabilitation mechanism (30) according to one of the preceding claims, **characterized in that** the extension arm (53) has a recess (55) engaging around the foot (94) of a leg (92).

8. Rehabilitation mechanism (30) according to one of the preceding claims, further comprising

   - a foot module (40) which can be operatively connected to the feet (94) and/or the soles (95) of the bedridden patient (90).

9. Rehabilitation mechanism (30) according to Claim 8, **characterized in that** the foot module (40) is designed such that a torque is applied to the ankle joint of the patient (90) during a rehabilitation movement of the patient (90).

10. Bed (10) for bedridden patients (90), comprising at least a rehabilitation mechanism (30) according to one of the preceding claims.

**Revendications**

1. Mécanisme de rééducation (30) pour un lit (10) comportant des côtés longitudinaux (12) s'étendant dans la direction y ainsi que des côtés transversaux (13) s'étendant dans la direction x, réalisé de manière appropriée pour une rééducation planifiée et automatisée au moins des articulations, des muscles et des tendons des jambes (92) d'un patient alité (90), comportant au moins :

   - un module pour genoux (50) pouvant être amené en liaison fonctionnelle avec les articulations de genou (93) du patient alité (90),
   - au moins un capteur d'angle (55), lequel surveille l'angle formé par un élément de liaison (52) par rapport à une orthèse de genou (51) et/ou par rapport à la potence (53) ; et
   - un module de commande (60) servant à obtenir des mouvements de rééducation planifiés au moins des articulations, des muscles et des tendons des jambes (92) du patient alité (90) au moyen du module pour genoux (50),

      - le module de commande (60) étant conçu pour, en cas de dépassement vers le haut ou vers le bas d'un seuil d'angle de consigne ($\varphi\_soll$) prédéfini de manière variable et surveillé par le capteur d'angle (55), commander un mécanisme (61) de telle sorte qu'un angle réel ($\varphi\_ist$) soit ramené au seuil d'angle de consigne ($\varphi\_soll$) en dessous

des seuils dépassés vers le haut ou au-dessus des seuils dépassés vers le bas par ajustement de disques excentriques (632, 642) ;

   **caractérisé en ce que**

   - le module pour genoux (50) comporte au moins :

      - une orthèse de genou (51) disposée au-dessus d'un matelas (20) dans la direction z pour la réception d'une articulation du genou (93) du patient alité (90) lors de l'utilisation, l'articulation du genou (93) étant en l'occurrence constamment à hauteur d'une articulation centrale de l'orthèse de genou (51) ;
      - un élément de liaison (52) relié à l'orthèse de genou (51) ;
      - une partie proximale (532) d'une potence (53) à laquelle l'élément de liaison (52) est fixé, laquelle partie proximale est disposée au-dessus du patient (90) et du matelas (20) dans la direction z,
      - et un mécanisme (61) pouvant être commandé au moyen du module de commande (60) pour le support direct ou indirect sur un lit (11) ou un cadre de matelas (21), lequel mécanisme, par le biais de la partie proximale (532) de la potence (53), de l'élément de liaison (52) et
      - d'un point de contact auquel la partie proximale (532) de la potence (53) est reliée à l'élément de liaison (52), et lequel se situe exactement au-dessus du centre de rotation de l'orthèse de genou (51), introduit une force définie (N) dans l'orthèse de genou (51) exactement au-dessus d'un centre de rotation de celle-ci dans la direction z, de telle sorte que, par le biais de l'articulation de genou (93) du patient alité (90), laquelle est reçue dans cette orthèse lors de l'utilisation, les articulations, les muscles et les tendons de cette jambe (92) réalisent des mouvements de rééducation planifiés,

   - le mécanisme (61) comportant deux excentriques (63, 64), lesquels sont formés respectivement par un disque excentrique (632, 642) monté sur un arbre à excentrique (631, 641), disque dont le centre se situe respectivement au-dessus de l'axe d'arbre ;
   - les arbres à excentriques (631, 641) étant entraînés par un moteur électrique (62) ; et
   - une partie distale (531) de la potence (53) étant reliée fonctionnellement à un boulon de commande (633) du premier disque excentrique (632) par le biais d'un palier radial (65) et à un boulon de commande (643) du deuxième disque excentrique (642) par le biais d'un palier lisse (66),

- et le module de commande (60) étant conçu pour déterminer le seuil d'angle de consigne (φ_soll) en fonction des angles d'excentrique alpha et bêta toujours de telle sorte que l'élément de liaison (52) entre la potence (53) et l'orthèse (51) soit constamment perpendiculaire au matelas (20).

2. Mécanisme de rééducation (30) selon la revendication 1, **caractérisé en ce que** le palier lisse (66) est formé par un coulisseau (67) relié de manière rotative au boulon de commande (643) du deuxième excentrique (64).

3. Mécanisme de rééducation (30) selon la revendication 1 ou 2, comportant en outre

- un capteur de force (57), lequel surveille la force (N) introduite dans l'orthèse de genou (51) par le biais de la potence (53) et de l'élément de liaison (52).

4. Mécanisme de rééducation (30) selon la revendication 3, **caractérisé en ce que** le module de commande (60) est conçu pour, en cas de dépassement vers le haut ou vers le bas d'un seuil de force de consigne prédéfini de manière variable et surveillé par le capteur de force (56), commander le mécanisme (61) de telle sorte que la force réelle (N) soit ramenée au seuil de force de consigne en dessous des seuils dépassés vers le haut ou au-dessus des seuils dépassés vers le bas par ajustement des disques excentriques (632, 642).

5. Mécanisme de rééducation (30) selon l'une des revendications précédentes, **caractérisé en ce que** le module de commande (60) est conçu pour commander le mécanisme (61) de telle sorte que l'ajustement des disques excentriques (632, 642) ait lieu séquentiellement ou en même temps que les mouvements de rééducation effectués de manière planifiée.

6. Mécanisme de rééducation (30) selon l'une des revendications précédentes, **caractérisé en ce que** la potence (53) comprend plusieurs points de réception (54) espacés les uns des autres, de préférence à des intervalles réguliers, pour le montage articulé de l'élément de liaison (52).

7. Mécanisme de rééducation (30) selon l'une des revendications précédentes, **caractérisé en ce que** la potence (53) comprend un évidement (55) enveloppant le pied (94) d'une jambe (92).

8. Mécanisme de rééducation (30) selon l'une des revendications précédentes, comportant en outre

- un module pour pieds (40) pouvant être amené en liaison fonctionnelle avec les pieds (94) et/les plantes de pieds (95) du patient alité (90).

9. Mécanisme de rééducation (30) selon la revendication 8, **caractérisé en ce que** le module pour pieds (40) est réalisé de telle sorte que, lors d'un mouvement de rééducation du patient (90), un couple est appliqué sur l'articulation de la cheville du patient (90).

10. Lit (10) pour patients alités (90), comportant au moins un mécanisme de rééducation (30) selon l'une des revendications précédentes.

# Fig. 1

# Fig. 2

# Fig. 3

# Fig. 4

Fig. 5

Fig. 6

Fig. 7

Fig. 8a

Fig. 8b

Fig. 8c

Fig. 9a

Fig. 9b

Fig. 9c

Fig. 10a

Fig. 10b

Fig. 10c

EP 3 362 023 B1

Fig. 11a     Fig. 11b     Fig. 11c

EP 3 362 023 B1

# Fig. 12

# Fig. 13

# Fig. 14

# Fig. 15

# Fig. 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0045897 A1
- WO 0061059 A1
- JP 2005334385 A
- JP H10258101 A
- JP 2003225264 A
- WO 2015158664 A1
- DE 4113135 A1
- US 20100042022 A1